# EUROPEAN PATENT SPECIFICATION

(11) **EP 2 140 844 B1**
(45) Date of publication and mention of the grant of the patent: **12.09.2012**
(21) Application number: 09164509.3
(22) Date of filing: 03.07.2009
(51) Int. Cl.: A61F 13/15, B32B 5/26, D04H 13/00

(54) **Nonwoven fibrous multiply fabric and method and plant for producing the fabric**
Mehrschichtiger Vliesstoff, Verfahren und Vorrichtung zu dessen Herstellung
Non-tissé multicouches, méthode et dispositif de fabrication d'un tel non-tissé

(30) Priority: 04.07.2008 DK 200800946
(43) Date of publication of application: 06.01.2010
(73) Proprietor: Oerlikon Textile GmbH & Co. KG, 42897 Remscheid (DE)
(72) Inventor: Andersen, Jens Ole Bröchner, 8660, Skanderborg (DK)
(74) Representative: Holme Nielsen, Marianne

(56) References cited:
- EP-A- 1 356 797
- WO-A-00/71790
- WO-A-00/74620

## Description

The invention relates to a method for producing a nonwoven fibrous multiply fabric of plies, which are dry-formed on a movable support.

Nonwoven fabrics have wide utility in many applications of which can be mentioned for example sanitary towels and wipes.

The market requires that the fabrics should have qualities like strength, absorbency and durability and also that the price of the fabrics should be competitive since such fabrics commonly are used only one or a few times after which they are disposed.

The desired properties of a nonwoven fibrous multiply fabric are achieved by composing the fabric of different fibres and materials.

A well known fabric, which is suitable to many applications thus is composed of a first ply consisting of a mixture of cellulose fibres and synthetic fibres, a second ply consisting of a mixture of cellulose fibres and super absorbent powder and a third ply consisting of cellulose fibres only.

This known fabric is commonly produced in a process where the three plies in succession are airlaid upon an endless wire mesh screen with the first ply uppermost and the third ply lowermost.

In the process the third ply is compacted between a first pair of rollers before in succession air laying the second and the first ply on the third ply. The compacting of the third ply between the first pair of rollers causes hydrogen bonds to arise between the cellulose fibres of said ply.

The plies then are calibrated between a second pair of rollers to form a coherent web after which the underside of the web are embossed by passing a third pair of rollers at temperatures between 80 - 180°C, the temperature of the roller on the side of the first ply being lower than the melting point of the synthetic fibres.

Subsequently the web is subjected to further treatments among which can be mentioned moistening, applying of latex and heating.

The heating of the web is causing thermo-bonds to be formed between the synthetic fibres of the first ply and also water to vaporize whereby the hydrogen bonds between the cellulose fibres disintegrate.

The hydrogen bonds between the cellulose fibres are re-formed by moistening the web once more and then calibrating the web between a fourth pair of rollers.

It is however a serious problem that super absorbent powder of the second ply trends to be squeezed out of the web when being calibrated between the second pair of rollers, whereby the wire would be contaminated so that proper operation of the plant is not more possible. The compacting of the web therefore needs to be light in this stage.

Since the web thus is compacted only light super absorbent powder easily will be squeezed out of the web during the succeeding embossing process. The super absorbent powder then will adhere to the rollers and make the embossing of the embossing roller unfit for use after which embossing of the web cannot more take place.

The embossing of the web is performed on the third ply placed on the underside of the web. The cellulose fibres of the third ply are in this stage not yet covered of a layer of e.g. latex. Such layer is applied only at a later stage of the process.

The consequence of the missing covering layer is that there during the embossing process is generated dust, which is polluting the environment.

A further problem is that the process is costly to carry out and that the plant for carrying out the process is complicated and expensive.

Other nonwoven multiply fabrics are disclosed in WO 00/71790, EP 1 356 797 and WO 00/74620.

The above-mentioned disadvantages of the prior art method for producing a nonwoven fibrous multiply fabric are according to the present invention remedied by,
in a first aspect of the invention providing a method by means of which a nonwoven fibrous multiply fabric can be produced to a low price,
in a second aspect of the invention providing a method by means of which a strong nonwoven fibrous multiply fabric can be produced,
in a third aspect of the invention providing a method by means of which a nonwoven fibrous multiply fabric with excellent absorbency properties can be produced,
in a fourth aspect of the invention providing a method by means of which embossing of a nonwoven fibrous multiply fabric safely and accurately can be carried out,
in a fifth aspect of the invention providing a method by means of which a nonwoven fibrous multiply fabric dust-free can be produced.
in a sixth aspect according to the invention providing an inexpensive nonwoven fibrous multiply fabric.
in a seventh aspect according to the invention providing an inexpensive embossed nonwoven fibrous multiply fabric.
in an eight aspect according to the invention providing a simple, inexpensive and effective functioning plant for producing an inexpensive embossed nonwoven fibrous multiply fabric.
in a ninth aspect according to the invention providing a simple, inexpensive and effective functioning plant for producing an inexpensive nonwoven fibrous multiply fabric by means of which the fabric dust-free can be embossed.

The novel and unique features of the invention whereby this is achieved consist in the fact that the method for producing a nonwoven fibrous multiplies fabric comprises the steps of forming a first ply, compacting the first ply between a first pair of rollers, forming a second ply on top of the first ply, compacting the plies between a second pair of rollers to form a coherent web, applying a layer of latex on top of the web, heating the web, moistening the web, embossing the web between a third pair of rollers and cooling the web.

This method is costsaving since the process comprises substantially fewer production steps than prior art methods of that kind.

The method can according to the invention be used to form a nonwoven fibrous multiply fabric of two ore more plies on an endless wire mesh screen whereby an efficient production is achieved.

The nonwoven fibrous multiply fabric can in an advantageous embodiment according to the invention be formed of a first ply of a mixture of cellulose fibres and synthetic fibres, a second ply of a mixture of cellulose fibres and super absorbent powder, and a third ply of cellulose fibres only.

The first ply of the mixture of cellulose fibres and synthetic fibres can according to the invention be compacted between the first pair of rollers with a pressure high enough to cause hydrogen bonds to arise between the cellulose fibres. In this way is the first ply and thereby the finished nonwoven fibrous multiply fabric too already in this stage imparted an excellent strength.

The relatively large compacting of the first ply also brings the synthetic fibres of the ply together in a compact structure requiring a smaller quantity of synthetic fibres than usual for such plies of nonwoven fibrous multiply fabric produced by means of prior art methods. The resulting nonwoven fibrous multiply fabric produced by means of the method according to the invention therefore becomes relatively inexpensive.

The three plies can, according to the invention, be compacted to a coherent web between the second pair of rollers by use of a relatively light pressure so that super absorbent powder is not squeezed out of the web and into the wire. Thereby is avoided that the mesh of the wire is filled with super absorbent powder, which would render the wire unfit for being used for the production of the nonwoven fibrous multiply fabric.

The layer of latex is, according to the invention, applied on top of the third ply of cellulose fibres before performing the embossing process, which therefore can be carried out without generating dust, which would have a harmful effect to the environment.

Since only a thin layer of latex is required for preventing such generation of dust during the embossing process the layer of latex applied on the third ply of cellulose fibres have such a thickness that a part of latex could penetrate into the third ply before heating of the web. So the third ply could be covered of a very thin layer of latex reducing the cost of the nonwoven fibrous multiply fabric. According to the invention the layer of latex on top of the third ply of cellulose fibres penetrates partially into the third ply, which comprise a part between 70% and 90% of cellulose fibres and a part between 10% and 30 % of latex.

The heating of the web, after having applied latex on top of the third ply of cellulose fibres of the nonwoven fibrous multiply fabric, has the effect that thermo-bonds between the synthetic fibres are formed.

The thermo-bonds are formed in large quantities, even if the percentage of synthetic fibres in the first ply is relatively little, owing to the fact that the fibres of the first ply already are brought close together in a compact structure with many contact points formed between the synthetic fibres during the passage of the first pair of rollers.

By means of the method according to the invention a strong nonwoven fibrous multiply fabric thus can be formed by adding smaller quantities of synthetic fibres to the mixture of the first ply than is the case with the prior art methods of this kind. In this way is advantageously obtained an inexpensive and at the same time strong fabric.

The heating of the web can, according to the invention, take place at temperatures between 140° and 200° C.

The heating also causes water in the web to vaporize whereby hydrogen bonds between the cellulose fibres disintegrate.

The hydrogen bonds between the cellulose fibres can according to the invention be re-formed by after the heating process moistening the web and then embossing the web between a third pair of rollers at temperatures between 80° and 180°.

The invention also relates to a nonwoven fibrous multiply fabric, comprising a first ply of a mixture of cellulose fibres and synthetic fibres, a second ply of a mixture of cellulose fibres and super absorbent powder and a third ply of cellulose fibres.

The novel and unique feature of the invention consist in the fact that only a relatively small quantity of synthetic fibres is used to form the fabric.

The contents of synthetic fibres in the first ply can according to the invention be between 5% and 30%, preferable between 12% and 18% whereby an inexpensive nonwoven fibrous multiply fabric is achieved.

The quality of the nonwoven fibrous multiply fabric can according to the invention be improved by means of a layer of latex on top of the third ply.

Said layer of latex can according to the invention penetrate partially into the third ply, which comprise a part between 70% and 90% of cellulose fibres and a part between 10% and 30 % of latex.

The invention furthermore relates to a plant for producing a nonwoven fibrous multiply fabric.

The novel and unique feature of the invention consist in the fact that the plant comprises at least one endless wire mesh screen, a first dry-forming stage for forming the first ply on the endless wire mesh screen, a first pair of rollers for compacting the first ply, a second dry-forming stage for forming the second ply on top of the first ply, a second pair of rollers to form a coherent web by compacting the plies between said rollers, a latex applying stage for applying latex to the web, a heating stage for heating the web, a moistening stage for moistening the web, a third pair of rollers for embossing the web on the side of the web with the third ply and a cooling stage for cooling the web.

This plant has a simple, inexpensive and effectively functioning construction with substantially fewer production stages than prior art plants of that kind.

The invention will be explained in greater detail below where further advantageous properties and example embodiments are described with reference to the drawings, in which
Fig. 1 shows separately the layers of a fragment of a cross section of a nonwoven fibrous multiply fabric according to the invention,
Fig. 2 shows the same with assembled layers and in embossed state,
Fig. 3 shows the same seen from above,
Fig. 4 shows schematically a first embodiment of a plant according to the invention, and
Fig. 5 shows schematically a second embodiment of a plant according to the invention.

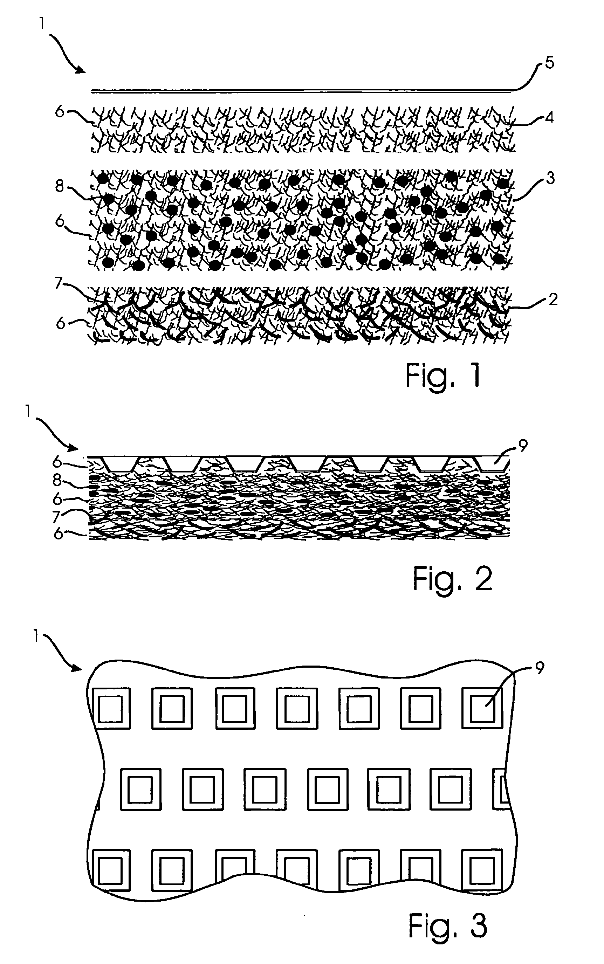

The nonwoven fibrous multiply fabric shown in fig. 1 - 3 is in its entirety designated with the reference numeral 1.

The fabric comprises in this case of four layers, which in fig. 1 are independently shown. The layers are a first ply 2, a second ply 3, a third ply 4 and a layer of latex 5.

The first ply consist, of a mixture of cellulose fibres 6 and synthetic fibres 7, the second ply of a mixture of cellulose fibres 6 and super absorbent powder 8 and the third ply of cellulose fibres 6 only.

The contents of synthetic fibres in the first ply is between 5% and 30%, preferable between 12% and 18% and the layer of latex on top of the third ply of cellulose fibres have penetrated partially into the third ply, which comprise a part between 70% and 90% of cellulose fibres and a part between 10% and 30 % of latex.

The contents of synthetic fibres in the first ply and the thickness of latex on top of the third ply could however in other embodiments have other values than stated above.

In fig. 2 the layers are assembled and compacted so they now form a coherent web.

The top of the web is, as seen in fig. 2 and 3, embossed with a number of pyramidal impressions 9 whereby the area of the surface of the web is increased and the absorption capacity of the fabric therefore is increased too. A desired pattern is at the same time imparted to the surface of the fabric.

The impressions can however, within the scope of the invention, have many other forms and be distributed in other ways for forming other desired patterns than shown in fig. 2 and 3.

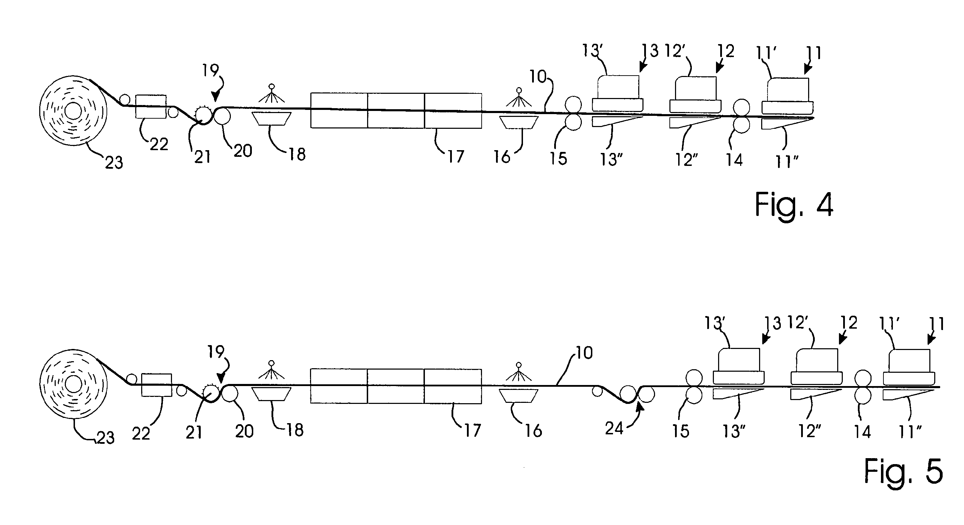

Fig. 4 shows a first embodiment of a plant for producing the nonwoven fibrous multiply fabric shown in fig. 1-3.

The fabric is transported from the right to the left on a wire mesh screen (not seen), which possibly could be divided up into more smaller wire mesh screens (not seen).

The nonwoven fibrous multiply fabric 1 is in all steps of the production indicated with the line 10.

The three plies 2, 3 and 4 are dry-formed on top of each other in three succeeding stages. The dry-forming technique can in itself be of any appropriate kind but is in the figure an air-laying technique performed by means of a first air-lying station 11, a second air-lying station 12 and a third air-laying station 13.

The air-lying stations 11, 12 and 13, which in the figure are shown only schematically, comprises each of a distributor box 11', 12' and 13', respectively placed above the wire and a suction box 11", 12" and 13" , respectively placed below the wire.

The fibres are during operation consecutively deposited in layers on the wire on top of each other in airflows between the distributor boxes and the suction boxes.

Instead of such air-laying stations can be used other types of dry-laying stations and instead of wire mesh screens can be used other types of moving supports.

The cellulose fibres 6 and synthetic fibres 7 of the first ply 2 are deposited in a layer on the wire by means of the first air-lying station 11.

The layer then is tight compacted by means of a heavy pressure when passing a first pair of rollers 14 having preferable smooth surfaces.

The heavy compacting of the first ply causes hydrogen bonds to be formed between the cellulose fibres with the result that the first ply obtains a relatively large strength.

The synthetic fibres of the first ply simultaneously are brought together in a tight structure requiring only a little quantity of synthetic fibres for being able to contribute to the desired strength of the ply and thereby of the resulting fabric.

The heavy compacting of the first ply therefore imparts large strength and low costs to the resulting fabric.

Afterwards the cellulose fibres 6 and the super absorbent powder 7 of the second ply 3 are deposited in a layer on the first ply 2 by means of the second air-lying station 12 after which the cellulose fibres 6 of the third ply are deposited in a layer on the second ply 3 by means of the third air-lying station 13.

The plies then are compacted between a second pair of rollers 15 to form a coherent web. The pressure used is moderate for avoiding that super absorbent powder is squeezed out of the web and is filling the meshes of the wire, which then not any more could operate in a proper way.

The surfaces of the rollers 15 are preferable smooth.

The layer of latex 5 is applied on top of the web in a latex applying station 16. The latex cans for example be sprayed upon the web.

The thickness of the layer of latex applied is of such a kind that even if a part of latex penetrates into the third ply the third ply will be covered completely with a thin layer of latex. This layer is thick enough to prevent dust to generate during the later embossing process. The layer is however so thin that the quantity of latex used is smaller than is the case with conventional plants of this kind whereby is obtained a further contribution to reducing the cost for producing the fabric.

The web now is heated in an heater 17 to temperatures in the interval 140° - 200° C whereby thermo-bonds between the synthetic fibres of the first ply of the nonwoven fibrous multiply fabric is formed.

The tight structure of the first ply formed by preliminary passing the first pair of rollers 14 secures many contact points between the synthetic fibres and thereby also that many thermo-bonds between the synthetic fibres is formed during the heating of the web by means of the heater 17.

The many thermo-bonds impart large strength to the first ply and thereby to the fabric even if only a reduced quantity of the relatively expensive synthetic fibres is used. Also in this way therefore is made a contribution to reducing the cost for producing the fabric.

The heating of the web to temperatures as high as between 140° - 200° C in the heater 17 also causes water in the web to vaporize and thereby the hydrogen bonds between the cellulose fibres to disintegrate.

The disintegrated hydrogen bonds between the cellulose fibres are re-formed by after the heating process moistening the web in a moistening station 18 and then compacting the web between a third pair of rollers 19 at temperatures between 80° and 180°.

The moistening of the web can take place by for example spraying or steaming the web.

The third pair of rollers consists of a roller 20 with a smooth surface and an embossing roller 21 with an embossed surface.

The embossing is performed on the side of the web with the third ply of cellulose fibres only. The thin layer of latex on this ply secure, as previously mentioned, that the embossing operation can be carried out without generating dust which otherwise could contaminate the environment.

The compacting and embossing of the web performed by means of the third pair of rollers 19 involves the risk that super absorbent powder could be squeezed out of the web and adhere to the hot rollers whereby the embossing of the embossing roller 21 would be made unfit for use.

The compacted first ply with the thermo-bonds between the synthetic fibres on one side of the web and the layer of latex on the other side of the web constitute however barriers which prevent the super absorbent powder to be squeezed out of the web.

The now hot web is cooled down in a cooling station 22 after which the finished fabric 10 is wound up on a reel 23.

Fig. 5 shows another embodiment for a plant according to the invention for producing the nonwoven fibrous multiply fabric shown in fig. 1 - 3.

This second embodiment of the plant corresponds to the first embodiment of the plant shown in fig. 4. Same reference numerals therefore are used for same parts.

In the first embodiment of the plant the three plies 2, 3 and 4 of the nonwoven fibrous multiply fabric were compacted or calendared by means of the second pair of rollers 15. The pressure used was moderate for preventing super absorbent powder to be squeezed out of the web and into the meshes of the wire mesh screen whereby the wire would be unfit for use. The plies therefore only are allowed a light compacting at this stage.

In the second embodiment of the plant therefore is inserted a fourth pair of rollers 24 between the second pair of rollers 15 and the latex applying station 16.

The web already light compressed by means of the moderate pressure performed by the second pair of rollers 15 now is further compressed with a pressure which similarly is moderate for also in this stage preventing super absorbent powder to be squeezed out of the web.

The resulting compression of the web is however higher than is possible by using only one pair of rollers.

It is noted that the above-mentioned operations within the scope of the invention can be supplied with other desired operations and treatments of the fabric.

## Claims

1. A method for producing a nonwoven fibrous multiply fabric of plies (1), which are dryformed on a movable support, **characterized in** comprising the steps of
forming a first ply (2),
compacting the first ply (2) between a first pair of rollers (14),
forming a second ply (3) on top of the first ply (2), the second ply (3) is formed of a mixture of cellulose fibres (6) and super absorbent powder (8).
compacting the plies (2,3,4) between a second pair of rollers (15) to form a coherent web (2,3,4),
applying a layer of latex (5) on top of the web (2,3,4),
heating the web (2,3,4),
moistening the web (2,3,4),
embossing the web (2,3,4) between a third pair of rollers (19), and
cooling the web (2,3,4).

2. A method according to claim 1, **characterized in that** a third ply (4) is formed on top of the second ply (3) before entering the second pair of rollers (15).

3. A method according to claim 1 or 2, **characterized in that** the first ply (2) is formed of a mixture of cellulose fibres (6) and synthetic fibres (7).

4. A method according to any of the claims 1 to 3, **characterized in that** the third ply (4) is formed of cellulose fibres (6).

5. A method according to any of the claims 1 to 4, **characterized in that** the first ply (2) is compacted between the first pair of rollers (14) with a pressure high enough to cause hydrogen bonds to arise between the cellulose fibres (6) of the first ply (2).

6. A method according to any of the claims 1 to 5, **characterized in that** the plies (2,3,4) are compacted to a coherent web (2,3,4) between the second pair of rollers (15) with a pressure, which is not so high that super absorbent powder is squeezed out of the web (2,3).

7. A method according to any of the claims 1 to 6, **characterized in that** the web (2,3,4) is heated to such a temperature after applying latex (5) on top of the web (2,3,4) that thermo-bonds are formed between the synthetic fibres (7) of the first ply (2).

8. A method according to any of the claims 1 to 7, **characterized in that** the top of the web (2,3,4) is embossed when passing the third pair of rollers (19).

9. A method according to any of the claims 1 to 8, **characterized in that** the web (2,3,4) is subjected to a temperature in the interval 80° to 180° C during the embossing process.

10. A method according to any of the claims 1 to 9, **characterized in that** that the web (2,3,4) is compacted between a fourth pair of rollers (24) after having passed the second pair of rollers (15).

11. A nonwoven fibrous multiply fabric (1) produced by means of the method according to claim 1 to 10, comprising a first ply (2) of a mixture of cellulose fibres (6) and synthetic fibres (7), a second ply (3) of a mixture of cellulose fibres (6) and super absorbent powder (8), and a third ply (4) of cellulose fibres (6), **characterized in that** the contents of synthetic fibres (7) in the first ply (2) is between 5% and 30%, preferable between 12% and 18%.

12. A nonwoven fibrous multiply fabric (1) according to claim 11, **characterized in that** the layer of latex (5) on top of the third ply (4) of cellulose fibres (6) penetrates partially into the third ply (4), which comprise a part between 70% and 90% of cellulose fibres and a part between 10% and 30 % of latex.

13. A plant according to the method of claim 1 to 12 and the nonwoven fibrous multiply fabric according to claim 11 and 12, **characterized in** comprising
at least one endless wire mesh screen,
a first dry-forming stage (11) for forming the first ply (2) on the endless wire mesh screen,
a first pair of rollers (14) for compacting the first ply (2),
a second dry-forming stage for forming the second ply (3) on top of the first ply (2),
a second pair of rollers (15) to form a coherent web (2,3,4) by compacting the plies (2,3,4) between said rollers (15),
a latex applying stage (16) for applying latex (5) to the web (2,3,4),
a heating stage (17) for heating the web (2,3,4),
a moistening stage (18) for moistening the web (2,3,4),
a third pair of rollers (19) for embossing the web (2,3,4) on the side with the third ply (4), and
a cooling stage (22) for cooling the web (2,3,4).

14. A plant according to claim 13, **characterized in** comprising a fourth pair of rollers (24) for compacting the web (2,3,4) in a stage after the second pair of rollers (15).

15. A plant according to claim 14, **characterized in that** the first, second and fourth pair of rollers (14,15,24) have smooth surfaces, while the third pair of rollers (19) has a roller (20) with a smooth surface and another roller (21) with an embossed surface.

## Patentansprüche

1. Verfahren zur Herstellung eines mehrlagigen Faservliesstoffs aus Lagen (1), die auf einem beweglichen Träger trockengeformt werden, **dadurch gekennzeichnet, dass** es die Schritte aufweist
Bilden einer ersten Lage (2),
Verdichten der ersten Lage (2) zwischen einem ersten Walzenpaar (14),
Bilden einer zweiten Lage (3) auf der ersten Lage (2), wobei die zweite Lage (3) aus einer Mischung von Zellulosefasern (6) und einem superabsorbierenden Pulver (8) gebildet wird,
Verdichten der Lagen (2,3,4) zwischen einem zweiten Walzenpaar (15) zur Bildung einer zusammenhängenden Bahn (2,3,4),
Auftragen einer Latexschicht (5) auf die Bahn (2,3,4),
Erwärmen der Bahn (2,3,4),
Befeuchten der Bahn (2,3,4),
Prägen der Bahn (2,3,4) zwischen einem dritten Walzenpaar (19), und
Abkühlen der Bahn (2,3,4).

2. Verfahren nach Anspruch 1, **dadurch gekennzeichnet, dass** auf der zweiten Lage (3) vor dem Eintritt in das zweite Walzenpaar (15) eine dritte Lage (4) gebildet wird.

3. Verfahren nach Anspruch 1 oder 2, **dadurch gekennzeichnet, dass** die erste Lage (2) aus einer Mischung von Zellulosefasern (6) und Kunstfasern (7) gebildet wird.

4. Verfahren nach einem der Ansprüche 1 bis 3, **dadurch gekennzeichnet, dass** die dritte Lage (4) aus Zellulosefasern (6) gebildet wird.

5. Verfahren nach einem der Ansprüche 1 bis 4, **dadurch gekennzeichnet, dass** die erste Lage (2) zwischen dem ersten Walzenpaar (14) mit einem Druck verdichtet wird, der hoch genug ist, um zu bewirken, dass zwischen den Zellulosefasern (6) der ersten Lage (2) Wasserstoffbindungen entstehen.

6. Verfahren nach einem der Ansprüche 1 bis 5, **dadurch gekennzeichnet, dass** die Lagen (2,3,4) zwischen dem zweiten Walzenpaar (15) mit einem Druck zu einer zusammenhängenden Bahn (2,3,4) verdichtet werden, der nicht so hoch ist, dass superabsorbierendes Pulver aus der Bahn (2,3) herausgequetscht wird.

7. Verfahren nach einem der Ansprüche 1 bis 6, **dadurch gekennzeichnet, dass** die Bahn (2,3,4) nach dem Auftragen von Latex (5) auf die Bahn (2,3,4) auf eine solche Temperatur erwärmt wird, dass thermische Bindungen zwischen den Kunstfasern (7) der ersten Lage (2) gebildet werden.

8. Verfahren nach einem der Ansprüche 1 bis 7, **dadurch gekennzeichnet, dass** die Oberseite der Bahn (2,3,4) geprägt wird, wenn sie das dritte Walzenpaar (19) passiert.

9. Verfahren nach einem der Ansprüche 1 bis 8, **dadurch gekennzeichnet, dass** die Bahn (2,3,4) während des Prägevorgangs einer Temperatur im Bereich von 80°C bis 180°C ausgesetzt wird.

10. Verfahren nach einem der Ansprüche 1 bis 9, **dadurch gekennzeichnet, dass** die Bahn (2,3,4) zwischen einem vierten Walzenpaar (24) verdichtet wird, nachdem sie das zweite Walzenpaar (15) passiert hat.

11. Mehrlagiger Faservliesstoff (1), der mittels des Verfahrens nach Anspruch 1 bis 10 hergestellt wird, der eine ersten Lage (2) aus einer Mischung von Zellulosefasern (6) und Kunstfasern (7), eine zweite Lage (3) aus einer Mischung von Zellulosefasern (6) und einem superabsorbierenden Pulver (8) und eine dritte Lage (4) aus Zellulosefasern (6) aufweist, **dadurch gekennzeichnet, dass** der Gehalt an Kunstfasern (7) in der ersten Lage (2) zwischen 5% und 30% und vorzugsweise zwischen 12% und 18% liegt.

12. Mehrlagiger Faservliesstoff (1) nach Anspruch 11, **dadurch gekennzeichnet, dass** die Latexschicht (5) auf der dritten Lage (4) aus Zellulosefasern (6) teilweise in die dritte Lage (4) eindringt, die einen Teil zwischen 70% und 90% Zellulosefasern und einen Teil zwischen 10% und 30% Latex aufweist.

13. Anlage gemäß des Verfahrens nach Anspruch 1 bis 12, und der mehrlagige Faservliesstoff nach Anspruch 11 und 12, **dadurch gekennzeichnet, dass** sie aufweist mindestens ein endloses Maschendrahtsieb,
eine erste Trockenformungsstufe (11) zum Bilden der ersten Lage (2) auf dem endlosen Maschendrahtsieb,
ein erstes Walzenpaar (14) zum Verdichten der ersten Lage (2),
eine zweite Trockenformungsstufe zum Bilden der zweiten Lage (3) auf der ersten Lage (2),
ein zweites Walzenpaar (15) zur Bildung einer zusammenhängenden Bahn (2,3,4) durch Verdichten der Lagen (2,3,4) zwischen den Walzen (15),
eine Latexauftragungsstufe (16) zum Auftragen von Latex (5) auf die Bahn (2,3,4),
eine Erwärmungsstufe (17) zum Erwärmen der Bahn (2,3,4),
eine Befeuchtungsstufe (18) zum Befeuchten der Bahn (2,3,4),
ein drittes Walzenpaar (19) zum Prägen der Bahn (2,3,4) auf der Seite mit der dritten Lage (4), und
eine Abkühlungsstufe (22) zum Abkühlen der Bahn (2, 3, 4).

14. Anlage nach Anspruch 13, **dadurch gekennzeichnet, dass** sie ein viertes Walzenpaar (24) zum Verdichten der Bahn (2,3,4) in einer Stufe nach dem zweiten Walzenpaar (15) aufweist.

15. Anlage nach Anspruch 14, **dadurch gekennzeichnet, dass** das erste, zweite und vierte Walzenpaar (14,15,24) glatte Oberflächen aufweisen, währen das dritte Walzenpaar (19) eine Walze (20) mit einer glatte Oberfläche und eine andere Walze (21) mit einer erhabenen Oberfläche aufweist.

## Revendications

1. Un procédé de production d'une structure multicouche fibreuse non tissée d'épaisseurs (1) qui sont formées à sec sur un support mobile, **caractérisé en ce qu'**il comprend les étapes suivantes :
formation d'une première épaisseur (2),
compactage de la première épaisseur (2) entre une première paire de rouleaux (14),
formation d'une seconde épaisseur (3) par-dessus la première épaisseur (2), la seconde épaisseur (3) étant formée d'un mélange de fibres de cellulose (6) et d'une poudre super-absorbante (8),
compactage des épaisseurs (2, 3, 4) entre une seconde paire de rouleaux (15) pour former une bande cohérente (2, 3, 4),
application d'une couche de latex (5) par-dessus la bande (2, 3, 4),
chauffage de la bande (2, 3, 4),
humidification de la bande (2, 3, 4),
gaufrage de la bande (2, 3, 4) entre une troisième paire de rouleaux (19), et
refroidissement de la bande (2, 3, 4).

2. Un procédé selon la revendication 1, **caractérisé en ce qu'**une troisième épaisseur (4) est formée par-dessus la seconde épaisseur (3) avant l'entrée dans la seconde paire de rouleaux (15).

3. Un procédé selon la revendication 1 ou 2, **caractérisé en ce que** la première épaisseur (2) est formée d'un mélange de fibres de cellulose (6) et de fibres synthétiques (7).

4. Un procédé selon l'une des revendications 1 à 3, **caractérisé en ce que** la troisième épaisseur (4) est formée de fibres de cellulose (6).

5. Un procédé selon l'une des revendications 1 à 4, **caractérisé en ce que** la première épaisseur (2) est compactée entre la première paire de rouleaux (14) avec une pression suffisamment élevée pour provoquer l'apparition de liaisons hydrogène entre les fibres de cellulose (6) de la première épaisseur (2).

6. Un procédé selon l'une des revendications 1 à 5, **caractérisé en ce que** les épaisseurs (2, 3, 4) sont compactées en une bande cohérente (2, 3, 4) entre la seconde paire de rouleaux (15) avec une pression qui n'est pas suffisamment élevée pour provoquer l'expulsion de la poudre super-absorbante hors de la bande (2, 3).

7. Un procédé selon l'une des revendications 1 à 6, **caractérisé en ce que** la bande (2, 3, 4) est chauffée à une température telle après application du latex (5) par-dessus la bande (2, 3, 4) que des thermoliaisons sont formées entre les fibres synthétiques (7) de la première épaisseur (2).

8. Un procédé selon l'une des revendications 1 à 7, **caractérisé en ce que** le dessus de la bande (2, 3, 4) est gaufré lors du passage dans la troisième paire de rouleaux (19).

9. Un procédé selon l'une des revendications 1 à 8, **caractérisé en ce que** la bande (2, 3, 4) est soumise à une température comprise dans l'intervalle allant de 80° à 180°C durant le processus de gaufrage.

10. Un procédé selon l'une des revendications 1 à 9, **caractérisé en ce que** la bande (2, 3, 4) est compactée entre une quatrième paire de rouleaux (24) après être passée par la seconde paire de rouleaux (15).

11. Une structure multicouche fibreuse non tissée (1) produite au moyen du procédé selon l'une des revendications 1 à 10, comprenant une première épaisseur (2) d'un mélange de fibres de cellulose (6) et de fibres synthétiques (7), une seconde épaisseur (3) d'un mélange de fibres de cellulose (6) et de poudre super-absorbante (8), et une troisième épaisseur (4) de fibres de cellulose (6), **caractérisée en ce que** la teneur en fibres synthétiques (7) dans la première épaisseur (2) est comprise entre 5 % et 30 %, de préférence entre 12 % et 18 %.

12. Une structure multicouche fibreuse non tissée (1) selon la revendication 11, **caractérisée en ce que** la couche de latex (5) par-dessus la troisième épaisseur (4) de fibres de cellulose (6) pénètre partiellement dans la troisième épaisseur (4), qui comprend une fraction comprise entre 70 % et 90 % de fibres de cellulose et une fraction comprise entre 10 % et 30 % de latex.

13. Une installation selon le procédé de la revendication 1 à 12 et pour la structure multicouche fibreuse non tissée de la revendication 11 et 12, **caractérisée en ce qu'**elle comprend :
au moins un tamis en grillage sans fin,
un premier étage de formage à sec (11) pour former la première épaisseur (2) sur le tamis en grillage sans fin,
une première paire de rouleaux (14) pour compacter la première épaisseur (2),
un second étage de formage à sec pour former la seconde épaisseur (3) par-dessus la première épaisseur (2),
une seconde paire de rouleaux (15) pour former une bande cohérente (2, 3, 4) par compactage des épaisseurs (2, 3, 4) entre lesdits rouleaux (15),
un étage d'application de latex (16) pour appliquer du latex (5) sur la bande (2, 3, 4),
un étage de chauffage (17) pour chauffer la bande (2, 3, 4),
un étage d'humidification (18) pour humidifier la bande (2, 3, 4),
une troisième paire de rouleaux (19) pour gaufrer la bande (2, 3, 4) du côté où se trouve la troisième épaisseur (4), et
un étage de refroidissement (22) pour refroidir la bande (2, 3, 4).

14. Une installation selon la revendication 13, **caractérisée en ce qu'**elle comprend une quatrième paire de rouleaux (24) pour compacter la bande (2, 3, 4) à un étage après la seconde paire de rouleaux (15).

15. Une installation selon la revendication 14, **caractérisée en ce que** les première, seconde et quatrième paires de rouleaux (14, 15, 24) présentent des surfaces lisses, tandis que la troisième paire de rouleaux (19) possède un rouleau (20) avec une surface lisse et un autre rouleau (21) avec une surface gaufrée.
